(19) Europäisches Patentamt
European Patent Office
Office européen des brevets

(11) Publication number: 0 219 470
A2

(12) **EUROPEAN PATENT APPLICATION**

(21) Application number: 86830291.0

(22) Date of filing: 09.10.86

(51) Int. Cl.[4]: A 61 K 31/545
C 07 D 501/36

(30) Priority: 15.10.85 IT 2248885

(43) Date of publication of application:
22.04.87 Bulletin 87/17

(84) Designated Contracting States:
AT BE CH DE FR GB LI LU NL SE

(71) Applicant: LARK S.p.A.
Via E. Romagnoli, 6
I-20146 Milan (IT)

(72) Inventor: Frare, Giovanni
Via G. Leopardi, 7
I-20050 Lesmo (Milan) (IT)

Brivio, Massimo
Via A. Moro, 21
I-22059 Robbiate (Como) (IT)

Terrassan, Daniele
Via Don Bosco, 1
I-20049 Concorezzo (Milan) (IT)

Biffi, Adriano
Via Resegone 6
I-22064 Casatenovo (Como) (IT)

(74) Representative: Perani, Aurelio et al
c/o JACOBACCI-CASETTA & PERANI S.p.A 7, Via
Visconti di Modrone
I-20122 Milano (IT)

(54) **Crystalline glycerol-solvated cefatrizine and method for its preparation.**

(57) The invention provides a new crystalline form of cefatrizine consisting of its glycerol-solvate which is stable in aqueous suspension and substantially free from impurities.

FIG. 2

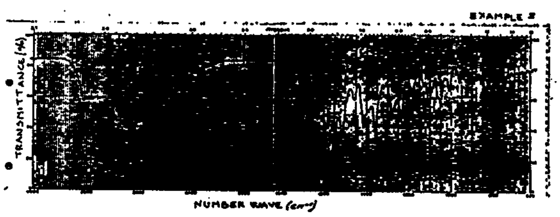


EP 0 219 470 A2

## Description

"Crystalline glycerol-solvated cefatrizine and method for its preparation"

The present invention relates to a new crystalline form of 7-(D-alpha-amino-alpha-(p-hydroxyphenyl)acetamido) -3-(1,2,3-triazol-5-ylthiomethyl)-3-cephem-4- carboxylic acid which will be referred to below by the international denomination cefatrizine.

Cefatrizine is an antibacterial agent which can be administered orally or parenterally and the preparation and pharmaceutical activity of which are described in U.S. Patent No. 3,855,213 and German Patent DE-PS 2,364,192.

It is known that the purification of crude cefatrizine is particularly difficult since the reaction product is highly contaminated by residues of the reagents. Moreover the molecule is unstable, being subject to degradation in water at alkaline pHs.

In order to purify the compound and provide a stable pharmaceutical form administrable in aqueous suspension, crystalline forms have been proposed which are constituted by solvates such as the methanolate, ethanolate, and the 1,2-propylene glycolate of cefatrizine and a hydrated form constituted by the crystalline sesquihydrate. Of these the only one which has been used effectively in commercial pharmaceutical forms is the 1,2-propylene glycolate of cefatrizine.

The subject of the present invention is a new crystalline form of cefatrizine which is stable in aqueous suspension and can be obtained in an extremely pure state substantially free from contaminants, and constituted by a glycerol solvate of cefatrizine. It has, in fact, been found that cefatrizine crystallises with from 1 to 1.5 moles of glycerol per mole of the cephalosporin. The melting point of the new crystalline form of glycerol-solvated cefatrizine is 185-193°C.

A further subject of the present invention is a method for the preparation of the crystalline glycerol-solvated cefatrizine which includes the operation of adding an organic base to an acidic solution of cefatrizine in aqueous glycerol until the pH of the solution is brought to a value of from 4.5 to 5 to precipitate the crystalline glycerol solvate and of recovering the solvate from the solution.

The base used in the method is preferably a tertiary amine, preferably triethylamine.

The acidic solution of cefatrizine used in the method of the invention may be obtained by the preparation of an aqueous suspension of crude cefatrizine in glycerol, the cefatrizine being amorphous or preferably one of its hydrates or solvates, such as the methanolate, ethanolate or 1,2-propylene glycolate, followed by the addition of a concentrated acid, preferably hydrochloric acid, until it has dissolved completely.

The methanolate, 1,2-propylene glycolate and ethanolate may in their turn be made by precipitation from an acidic solution of crude amorphous cefatrizine in methanol, 1,2-propylene glycol and ethanol respectively by the addition of an organic base.

Example 1

50g of cefatrizine solvated with 1,2-propylene glycol are suspended in 350 ml of a 65% v/v glycerol/water solution at 18-20°C.

Concentrated hydrochloric acid is added until dissolution is complete at a pH of about 0.5. 5g of decolourising carbon are then added and the suspension is agitated for 30-45 minutes. The suspension is then filtered and the filter cake washed with 50 ml of the 65% glycerol/water solution. Triethylamine is then added drop by drop over about 1 hour to bring the pH to 4.5-4.8. During this phase the glycerol- solvated cefatrizin starts to precipitate, the precipitation starting at a pH of from 2.6 to 2.8. After 2 hours of agitation at pH 4.5-4.8 and 18°C the crystalline solvate is recovered by filtration, washed with 100 ml of a 70% v/v glycerol/water solution, 100 ml of ethanol and 100 ml of acetone.

The glycerol-solvated cefatrizine is dried at 50°C s.v. for about 10 hours. A 95% yield is obtained. The solvate obtained is identified as the glycerol solvate and quantified by HPLC against a standard of cefatrizine 1,2-propylene glycolate.

HPLC titre 76.5-77.5%

Glycerol content 20-21%

The infra-red spectrum of the glycerol-solvated cefatrizine obtained, determined in 1% concentration in KBr pellets, is illustrated in Figure 1.

Example 2

50g of methanol-solvated cefatrizine are suspended in 350 ml of a 52% v/v glycerol/water solution at 18-20°C.

Concentrated hydrochloric acid is added until dissolution is complete at a pH of about 0.5. The pH is corrected to 1.5-1.6 by the addition of triethylamine. 5g of decolourising carbon are added and the suspension is agitated for 30-45 minutes. The carbon is filtered off, the filter cake is washed with 50 ml of a 52% v/v glycerol/water solution. The pH of the decolourised solution is corrected to 4.5-4.8 by the slow addition of triethylamine over about 1 hour. During this phase, starting at a pH of 2.6-2.8, the glycerol-solvated cefatrizine precipitates. The mixture is kept under agitation at pH 4.5-4.8 for two hours and then the crystals are recovered by filtration. They are washed with 100 ml of 60% v/v glycerol/water solution, 100 ml of ethanol and 100 ml of acetone and dried at 50°C s.v. for 10-12 hours.

Yield: 94%

HPLC titre 81-82% as cefatrizine

Glycerol content 16-17%

The infra-red spectrum of the glycerol-solvated cefatrizine obtained, determined in 1% concentration in KBr pellets, is given in Figure 2.

Example 3

50g of hydrated cefatrizine are suspended in 350 ml of a 50% glycerol/water solution at a temperature of 18-20°C.

Concentrated hydrochloric acid is added until

dissolution is complete at a pH of about 1. 5g of decolourising carbon are added and the whole is agitated for 30-45 minutes. The suspension is then filtered, the cake washed with 50ml of 50% glycerol/water solution and the washings added to the filtrate. The pH of the solution is then corrected to 4.5-4.8 by the addition of triethylamine over about 1 hour. After about 2 hours from the end of the addition of triethylamine, the precipitate if filtered, washed with 100 ml of a 60% glycerol/water solution, 100 ml of ethanol and 100 ml of acetone and dried at 50°C s.v. for 10-12 hours.

Yield: 90%

HPLC titre 82-83% as cefatrizine

Glycerol content 15-17%

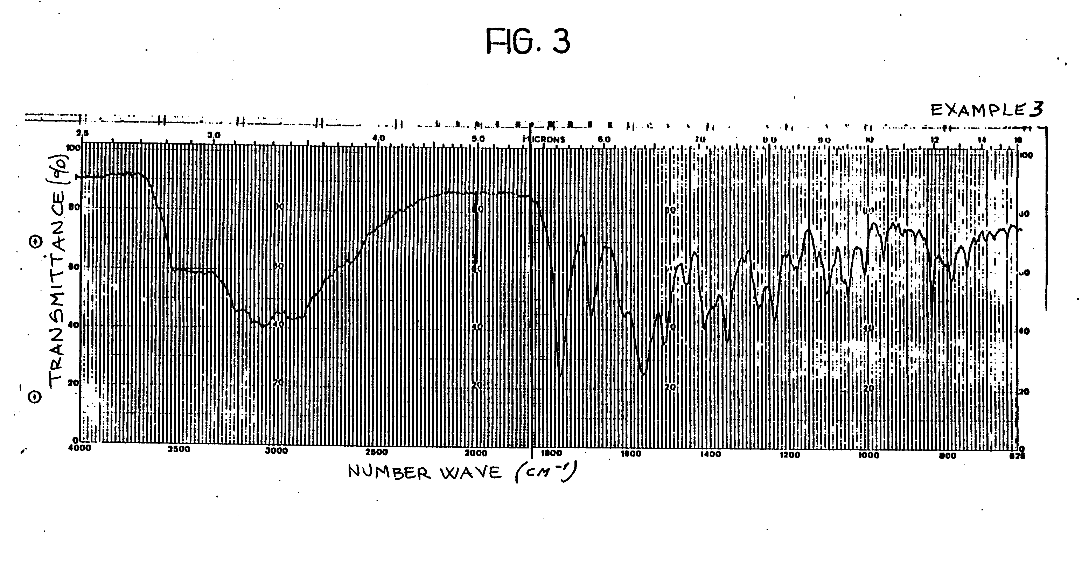

The infra-red spectrum of the glycerol-solvated cefatrizine obtained, determined in 1% concentration in KBr pellets, is illustrated in Figure 3.

The crystalline glycerol solvate is substantially free from impurities which are normally found in cefatrizine obtained by commercial industrial processes. When suspended in water it does not lose its biological activity and crystalline properties. The dried solid may also be kept for long periods of time without substantial reduction in its biological activity.

The glycerol-solvate thus lends itself to oral administration in aqueous suspension or to administration in the form of capsules or tablets containing the solid product for oral use.

**Claims**

1. Crystalline glycerol-solvated cefatrizine (melting point: 185-193°C).

2. Crystalline glycerol-solvated cefatrizine having from 1 to 1.5 moles of glycerol per mole of cephalosporin.

3. Method for the preparation of crystalline glycerol-solvated cefatrizine, characterized in that it includes the operation of adding an organic base to an aqueous acidic solution of cefatrizine in glycerol until the pH of the solution has reached a value of from 4 to 5 to precipitate the crystalline solvate, and of recovering the solvate from the solution.

4. Method according to Claim 3, in which the base is a tertiary amine.

5. Method according to Claim 4, in which the base is triethylamine.

6. Method according to one of Claims 3 to 5, in which the aqueous, acidic solution of cefatrizine in glycerol is acidified with hydrochloric acid to a pH of 0.5-1.

7. Pharmaceutical composition having antibacterial activities, containing a therapeutically effective quantity of glycerol-solvated cefatrizine.

FIG. 1

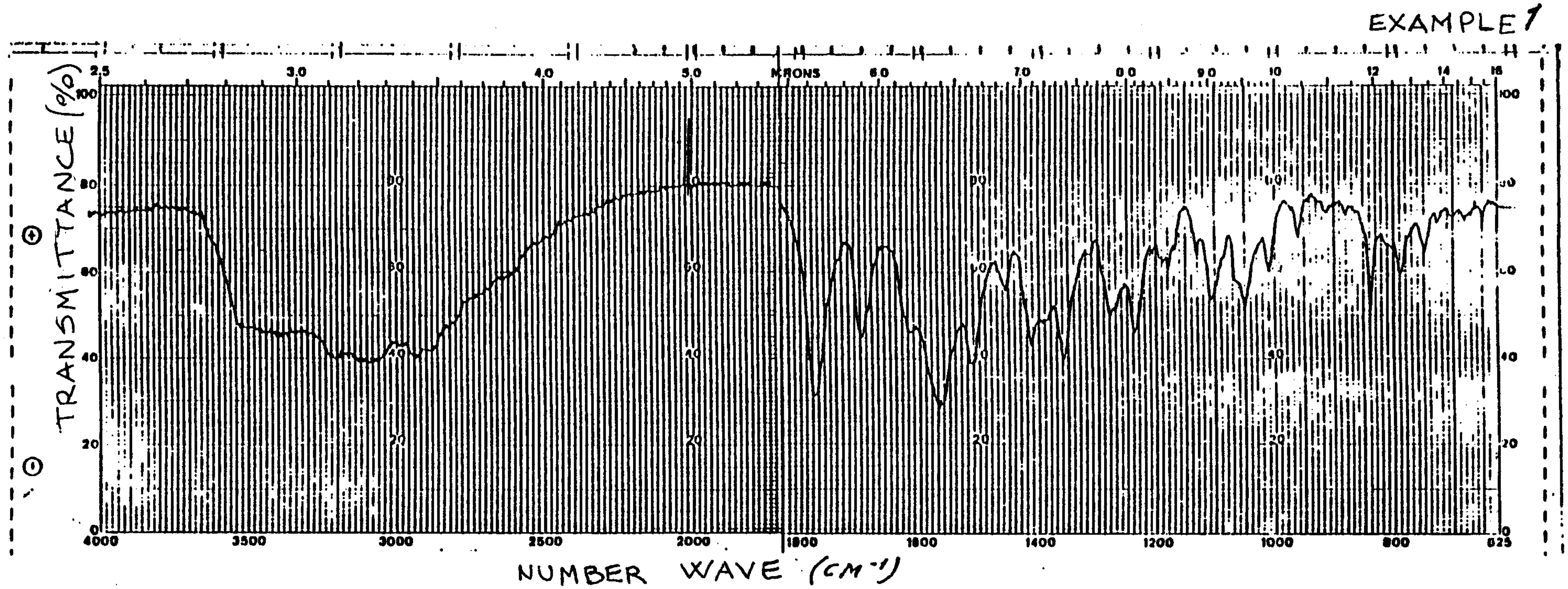

FIG. 2

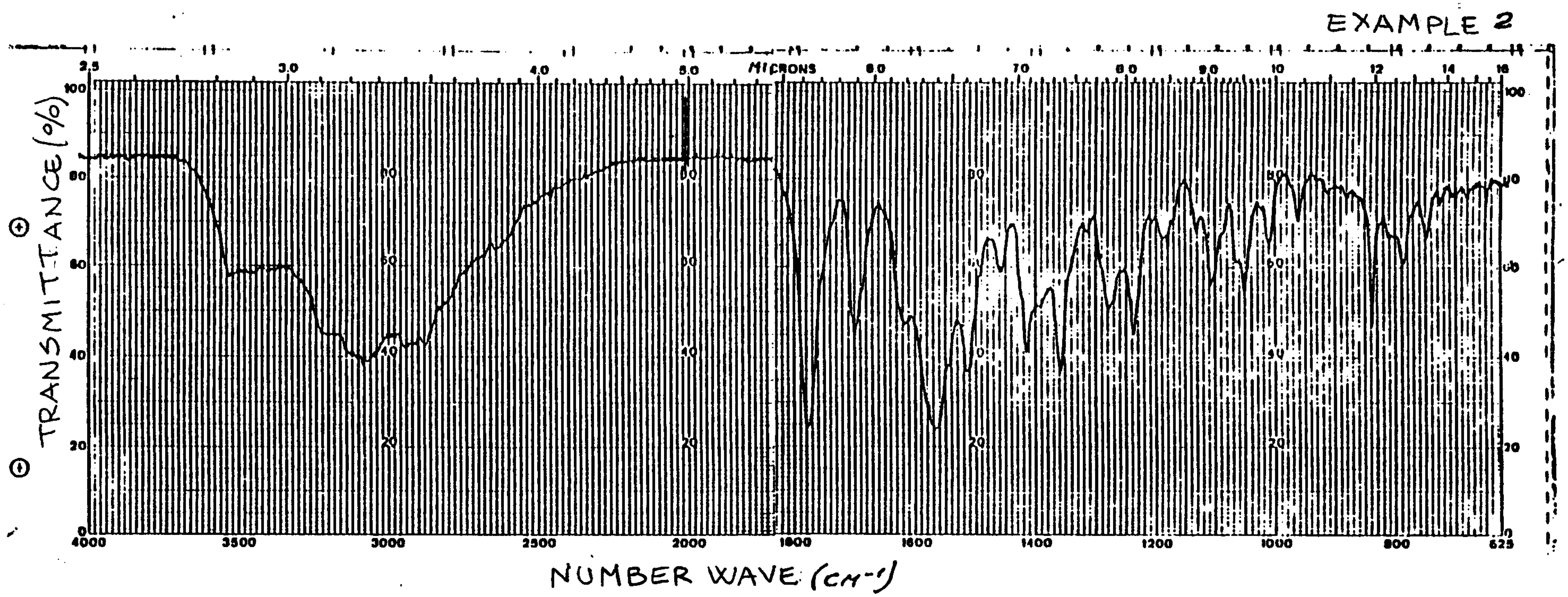
EXAMPLE 2
MICRONS
2.5
3.0
4.0
5.0
6.0
7.0
8.0
9.0
10
12
14
16
TRANSMITTANCE (%)
100
80
60
40
20
0
4000
3500
3000
2500
2000
1800
1600
1400
1200
1000
800
625
NUMBER WAVE (CM⁻¹)

FIG. 3